(19) Europäisches Patentamt
European Patent Office
Office européen des brevets

(11) Veröffentlichungsnummer: **0 280 868 B1**

(12) # EUROPÄISCHE PATENTSCHRIFT

(45) Veröffentlichungstag der Patentschrift: **17.06.92**

(21) Anmeldenummer: **88101056.5**

(22) Anmeldetag: **26.01.88**

(51) Int. Cl.⁵: **C07D 513/04**, C07D 233/86, C07D 233/54, A61K 31/54, //(C07D513/04,285:00,235:00), (C07D513/04,285:00,249:00)

(54) **Neue Imidazo- und Triazolothiadiazine, Verfahren zu ihrer Herstellung, die sie enthaltenden Arzneimittel und ihre Verwendung, sowie einige bei der Herstellung der genannten Verbindungen gebildete Zwischenprodukte.**

(30) Priorität: **30.01.87 DE 3702757**

(43) Veröffentlichungstag der Anmeldung:
**07.09.88 Patentblatt 88/36**

(45) Bekanntmachung des Hinweises auf die Patenterteilung:
**17.06.92 Patentblatt 92/25**

(84) Benannte Vertragsstaaten:
**AT BE CH DE ES FR GB GR IT LI LU NL SE**

(56) Entgegenhaltungen:
**DE-A- 1 620 071**

**CHEMICAL ABSTRACTS, Band 98, Nr. 17, 25. April 1983, Seite 578, Zusammenfassung-Nr. 143384n, Columbus, Ohio, US; M.A. EL-DAWY et al.: "Potential broad spectrum anthelmintics IV: design, synthesis, and antiparasitic screening of certain 3,6-disubstituted-(7H)-s-triazolo(3,4-b)(1,3,4)-thiadiazine derivatives"**

(73) Patentinhaber: **HOECHST AKTIENGESELL-SCHAFT**
**Postfach 80 03 20**
**W-6230 Frankturt am Main 80(DE)**

(72) Erfinder: **Thorwart, Werner, Dr.**
**Am Gänsborn 3b**
**W-6203 Hochheim am Main(DE)**
Erfinder: **Gebert, Ulrich, Dr.**
**Albert-Lortzing-Strasse 2**
**W-6233 Kelkheim (Taunus)(DE)**
Erfinder: **Schleyerbach, Rudolf, Dr.**
**Finkenweg 10**
**W-6238 Hofheim am Taunus(DE)**
Erfinder: **Bartlett, Robert R., Dr.**
**Sandbergstrasse 20**
**W-6100 Darmstadt(DE)**

EP 0 280 868 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

CHEMICAL ABSTRACTS, Band 90, Nr. 5, 29. Januar 1979, Seite 486, Zusammenfassung-Nr. 38886q, Columbus, Ohio, US; S. BALA et al.: "Heterocyclic systems containing bridgehead nitrogen atoms: part XXXIII. Syntheses of s-triazolo(3,4-b)(1,3,4)thiadiazine, s-triazolo(3,4-b)(1,3,4)triadiazino(6,7-b)quinoxaline and as-triazino(3,4-b)(1,3,4)thiadiazines"

ZEITSCHRIFT FUER CHEMIE, Band 15, Nr. 12, 1975, Seite 482; E. BULKA et al.: "Ueber die Synthese von 2H-Imidazo(2,1-b)-(1,3,4)-thiadiazinen"

CHEMICAL ABSTRACTS, Band 70, Nr. 21, 26. Mai 1969, Seite 347, Zusammenfassung-Nr. 96771w, Columbus, Ohio, US; G. WESTPHAL et al.: "Preparation of some 7H-s-triazolo(3,4-b)(1,3,49-thiadiazines"

CHEMICAL ABSTRACTS, Band 59, Nr. 5, 2. September 1963, Spalten 5159f, 5160d-5161c, Columbus, Ohio, US; T. PYL et al.: "Bicyclic heterocyclic compounds with a common nitrogen atom. VI. Formation of 1-amino-2-mercaptoimidazoles from 2-benzyl-thioimidazo(2,1-b)(1,3,4)-thiadiazoles"

CHEMICAL ABSTRACTS, Band 104, Nr. 7, 17. Februar 1986, Seite 513, Zusammenfassung-Nr. 50789a, Columbus, Ohio, US

CHEMICAL ABSTRACTS, Band 90, Nr. 15, 9. April 1979, Seite 28, Zusammenfassung-Nr. 114920p, Columbus, Ohio, US; H. VAN DER GOOT et al.: "The synthesis and anti-inflammatory activity of substituted 2-(4-hydroxyphenyl)-1,3-indandiones"

## Beschreibung

Die vorliegende Erfindung betrifft neue Imidazo- und Triazolothiadiazine, Verfahren zu ihrer Herstellung und ihre Verwendung als Wirkstoffe in Arzneimitteln zur Behandlung entzündlicher, insbesondere entzündlich rheumatischer Erkrankungen; außerdem bezieht sich die Erfindung auch auf einige bei der Herstellung der vorerwähnten Imidazo- und Thiazolo-thiadiazine gebildete Zwischenprodukte.

Bei den bisher in der Rheumatherapie vorzugsweise eingesetzten nicht-steroidalen Antiphlogistika handelt es sich fast ausschließlich um verhältnismäßig starke Cyclooxygenase-Inhibitoren, die den endogenen Abbau der Arachidonsäure zu den entzündungs- und schmerzfördernden Prostaglandinen hemmen. Allerdings stehen im ursächlichen Zusammenhang mit einer zu starken Hemmung der Cyclooxygenase-Aktivität eine Reihe von gravierenden Nebenwirkungen, wie gastrointestinale Beschwerden, Nierenfunktionsstörnungen und allergische Reaktionen (z.B. Hautallergien und Asthmaanfälle), die insbesondere bei der üblicherweise notwendigen Langzeitbehandlung häufig zum Abbruch der Therapie zwingen (vergl. K. Brune, Eur. J. Rheumatol. Inflam. 5 (1982), S. 335 - 349).
Ein weiterer mit dem beschriebenen Wirkungsmechanismus ursächlich verbundener Nachteil dieser klassischen nichtsteroidalen Antiphlogistika besteht darin, daß sie zwar die Beseitigung oder Linderung der Symptome Schmerz, Entzündung und Schwellung gestatten, aber die den entzündlich rheumatischen Erkrankungen zugrundeliegenden immunpathologischen Prozesse unbeeinflußt lassen und daher den progredienten Krankheitsverlauf nicht aufzuhalten vermögen.

Es besteht somit ein dringendes Bedürfnis an therapeutisch nutzbaren Antirheumatika, die sich aufgrund eines günstigeren Wirkungsprofils von den bekannten nichtsteroidalen Antiphlogistika vorteilhaft durch bessere Verträglichkeit einerseits und einen mehr kausalen Eingriff in das rheumatische Krankheitsgeschehen andererseits unterscheiden. Erfolgversprechende Ansatzpunkte hierfür bieten solche Pharmaka, die verstärkt in den alternativen Weg des Arachidonsäureabbaus eingreifen, indem sie beispielsweise die 5-Lipoxygenase hemmen und somit die exzessive Bildung der proinflammatorischen Leukotriene unterbinden, hochreaktive Sauerstoffradikale, die als Entzündungsmediatoren die Zell- und Gewebszerstörung in den entzündlich rheumatischen Gelenken perpetuell unterhalten, desaktivieren und/oder das entgleiste Immunsystem restaurieren und damit die Möglichkeit eröffnen, mehr ursächlich die rheumatischen Erkrankungen medikamentös zu behandeln.

Überraschend wurde nun gefunden, daß durch Einführung bestimmter 3-Alkyl-5-tert.butyl-4-hydroxyphenyl-Reste in die 3-Stellung von gegebenenfalls in 2-Position substituierten 2H-Imidazo[2,1-b]-[1,3,4]thiadiazinen und 2H-s-Triazolo[3,4-b][1,3,4]thiadiazinen neuartige Verbindungen erhalten werden, die aufgrund ihrer pharmakologischen Eigenschaften die zuvor aufgestellten Anforderungen erfüllen und sich demnach hervorragend zur Behandlung rheumatischer Erkrankungen eignen.
Die auch gastral äußerst gut verträglichen Verbindungen hemmen im Unterschied zu den bekannten nichtsteroidalen Antiphlogistika das Arachidonsäure-abbauende Enzym 5-Lipoxygenase, während eine Beeinflussung der Cyclooxygenase nicht nachgewiesen werden kann. Die Fähigkeit der Verbindungen, Sauerstoffradikale zu desaktivieren, zeigt sich beispielsweise im Modell der ®Adriamycin (Fa. Farmitalia)induzierten Entzündung und durch die Inhibierung der Lipidperoxydation.

Darüber hinaus greifen sie vorteilhaft in das gestörte Immunsystem ein, wie sich durch die Normalisierung der supprimierten Immunaktivität in den pathologischen Modellen der mit Freundschem Adjuvans oder Typ II-Collagen induziertem Arthritis demonstrieren läßt.

Bei den in der Literatur bereits beschriebenen 2H-Imidazo[2,1-b] [1,3,4]thiadiazinen handelt es sich durchweg um 3,7-diphenylierte Derivate, von denen keine pharmakologischen Eigenschaften bekannt sind (Th. Pyl, Fr. Waschk und H. Beyer, Liebigs Ann. Chem. 663 (1963), S. 113 - 119 und E. Bulka und W. D. Pfeiffer, Z. Chem. 15 (1975), S. 482). Von demselben Ringsystem leiten sich die partiell hydrierten 6,7-Dihydro-2H-imidazo[2,1-b] [1,3,4]thiadiazine und deren 6- bzw. 7-Oxo-Derivate ab, die bislang in der Literatur noch nicht beschrieben wurden und deren Derivate mit 3-ständigem 3-Alkyl-5-tert.butyl-4-hydroxyphenyl-Rest von vorliegender Erfindung mitumfaßt werden. Von den strukturell nahe verwandten 2H-s-Triazolo[3,4-b][1,3,4]thiadiazinen sind ebenfalls Diphenyl-Abkömmlinge bekannt (M. A. El-Dawy et al., J. Pharm. Sci. 72 (1983), S. 45 - 50), die anthelmintische Aktivität besitzen sollen.

Die vorliegende Erfindung betrifft demgegenüber neue 2H-Imidazo[2,1-b][1,3,4]thiadiazine und 2H-s-Triazolo[3,4-b] [1,3,4]thiadiazine, die in 3-Stellung einen 3-Alkyl-5-tert. butyl-4-hydroxyphenyl-Rest als essentielle pharmakophore Gruppe und gegebenenfalls in 2-Position einen weiteren Substituenten tragen. Aufgrund ihrer zuvor erwähnten pharmakologischen Eigenschaften eignen sich die erfindungsgemäßen Verbindungen zur Verwendung in Arzneimitteln, insbesondere in solchen, die bei entzündlich rheumatischen Erkrankungen indiziert sind.

Gegenstand der Erfindung sind somit neue Imidazo- und Triazolo-thiadiazine der allgemeinen Formel I,

in der

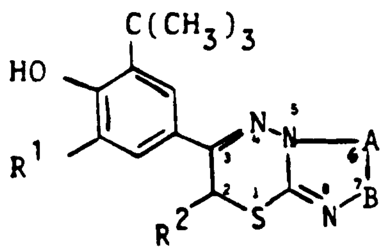

(I)

| | |
|---|---|
| $R^1$ | eine geradkettige oder verzweigte Alkylgruppe mit 1 bis 4-C-Atomen und |
| $R^2$ | ein Wasserstoffatom oder einen Alkylrest mit 1 bis 3 C-Atomen bedeuten und das zweigliedrige Strukturelement |
| -A-B- | im Fünfring für $-CH_2-CH_2-$, -CH = CH-, -CH = N-, $-CH_2$-CO-oder -CO-$CH_2$- steht, |

sowie deren physiologisch verträglichen Säureadditionssalze.

Bevorzugt sind dabei solche Verbindungen der Formel I, bei denen das Strukturelement -A-B- eine Äthylen- oder Vinylengruppe bedeutet oder aber entweder A oder B für eine Carbonylgruppe und das andere Brückenglied für eine Methylengruppe stehen. Unter diesen Verbindungen sind wiederum jene hervorzuheben, in denen $R^1$ eine tert.Butylrest bedeutet und $R^2$ für Wasserstoff oder Methyl steht, wie etwa das 3-(3,5-Di-tert.butyl-4-hydroxyphenyl)-6,7-dihydro-2H-imidazo[2,1-b][1,3,4]thiadiazin.

Als Alkylreste für die Gruppe $R^1$ kommen Methyl, Äthyl, n-Propyl, Isopropyl, n-Butyl, Isobutyl, sek. Butyl und tert.Butyl und für die Gruppe $R^2$ Methyl, Äthyl, n-Propyl und Isopropyl in Frage.

Gegenstand der Erfindung ist ferner ein Verfahren zur Herstellung der neuen Imidazo- und Triazolothiadiazine und ihrer physiologisch verträglichen Säureadditionssalze, darin bestehend, daß man ein 2-Halogen-1-phenylalkanon der Formel II,

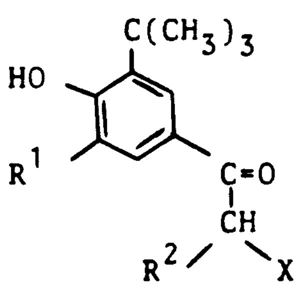

(II)

in der $R^1$ und $R^2$ die oben angegebene Bedeutung haben und X für ein Halogenatom, vorzugsweise Chlor oder Brom steht, mit einer Verbindung der Formel III,

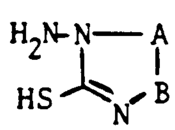

(III)

in der das zweigliedrige Strukturelement -A-B- die oben genannten Bedeutungen hat, zu den erfindungsgemäßen Verbindungen der Formel I umsetzt und diese entweder in freier Form isoliert oder mit geeigneten Säuren in physiologisch verträgliche Additionssalze umwandelt.

Für die Herstellung von Säureadditionssalzen kommen beispielsweise Mineralsäuren, wie Bromwasserstoff-, Chlorwasserstoff-, Schwefel- oder Phosphorsäure; organische Säuren, wie Essig-, Milch-, Malein-, Fumar-, Oxal-, Wein-, Zitronen- oder Glukonsäure; oder andere physiologisch verträgliche Säuren, wie Sulfonsäuren, z. B. Benzolsulfonsäure, p-Toluolsulfon-, Methansulfon-, Trifluormethylsulfon- und Cyclohexylamidosulfonsäure, in Frage.

Die für das erfindungsgemäße Verfahren als Ausgangsstoffe verwendeten 2-Halogen-1-phenylalkanone der Formel II sind literaturbekannt oder lassen sich aus den 1-(3-Alkyl-5-tert.butyl-4-hydroxyphenyl)-alkanonen durch Umsetzung mit einem geeigneten Halogenierungsmittel nach den im Houben-Weyl Bd. V/4 (1960), S. 171-189 beschriebenen Methoden leicht herstellen. Als geeignete Verbindungen II seien beispielsweise das 2-Brom-1-(3,5-di-tert.butyl-4-hydroxyphenyl)-äthanon und 2-Brom-1-(5-tert.butyl-3-methyl-4-hydroxyphenyl)-äthanon genannt, die sich durch Halogenierung der entsprechend substituierten 1-Phenylalkanone mit elementarem Brom oder mit Kupfer-II-bromid nach einem Verfahren von L. C. King und G. K. Ostrum, J. org. Chem. 29 (1964), S. 3459 - 3461 herstellen lassen.
Zur Gewinnung jener Verbindungen der Formel II,in denen für X ein Chloratom steht, eignet sich insbesondere Sulfurylchlorid, das vorzugsweise bei Temperaturen zwischen etwa 10 und 30 ° C in Anwesenheit inerter Lösungsmittel, wie z.B. Methylenchlorid oder Chloroform, mit den entsprechenden 1-Phenylalkanonen zur Reaktion gebracht wird. Ein weiteres Herstellungsverfahren besteht in der Friedel-Crafts-Acylierung von 2-Alkyl-6-tert.butylphenolen mit vorzugsweise Chloracetylchlorid in Gegenwart von Lewissäuren, wie z. B. Aluminiumchlorid oder Bortrifluorid.

Von den ebenfalls als Ausgangsstoffe eingesetzten Verbindungen der Formel III sind die folgenden Heterocyclen literaturbekannt:
1-Amino-2-thioxo-imidazolidin (K. H. Mayer und S. Petersen, Synthesis 1971, S. 370 -373), 4-Amino-3-mercapto-1,2,4-triazol (H. Beyer und C. F. Kröger, Liebigs Ann. Chem. 637 (1960), S. 135 -145) und 1-Amino-2-thioxo-4-imidazolidinon (H. Shirai und T. Yashiro, Yakugaku Zasshi 87 (1967), S. 137 - 142).
Neu hingegen ist das 1-Amino-2-mercapto-imidazol, das sich aus Thioisocyanatoacetaldehyd-dialkylacetalen und Hydrazin bevorzugt unter salzsauren Bedingungen in Lösungsmitteln,wie Methanol oder Äthanol, bei Reaktionstemperaturen unterhalb etwa 30 ° C leicht herstellen läßt.
Die alkalische Cyclisierung von Thiosemicarbazido-(4)-essigsäurealkyl- vorzugsweise -äthylester mit Natriumalkoholat liefert das ebenfalls neue 1-Amino-2-thioxo-5-imidazolidinon.

Die Umsetzung der 2-Halogen-1-phenylalkanone II mit Verbindungen der Formel III wird zweckmäßig mit äquimolaren Mengen der beiden Reaktanten in einem gegenüber den Reaktionspartnern inerten Lösungs- oder Verteilungsmittel durchgeführt. Hierfür kommen insbesondere polare Solventien, beispielsweise niedrige aliphatische Alkohole, wie Methanol, Äthanol, die verschiedenen Propanole oder Butanole, oder niedrige aliphatische Carbonsäuren, wie Essigsäure, sowie Mischungen der genannten Lösungsmittel oder deren Gemische mit Wasser in Frage. Es können aber auch Äthylenglykol und dessen Äther, Essigsäureester, Aceton, Butan-2-on, Dimethylformamid oder Acetonitril Verwendung finden. Die Reaktionstemperaturen liegen im allgemeinen zwischen etwa 20 ° C und dem Siedepunkt des jeweils verwendeten Reaktionsmediums. Vorzugsweise wird beim Arbeiten in Äthanol auf Siedetermperatur und in Essigsäure auf Temperaturen zwischen etwa 70 und 100 ° C erhitzt, wobei die Reaktionszeiten zwischen weniger als einer Stunde und etwa 12 Stunden liegen.

Die erfindungsgemäßen Imidazo- und Triazolo-thiadiazine der Formel I und ihre physiologisch verträglichen Säureadditionssalze eignen sich aufgrund ihrer wertvollen pharmakologischen Eigenschaften bei gleichzeitig hervorragender Verträglichkeit in besonderer Weise für die Verwendung als Wirkstoffe in Arzneimitteln, insbesondere in solchen zur Behandlung von entzündlich rheumatischen Erkrankungen. Sie können entweder für sich allein, beispielsweise in Form von Mikrokapseln, in Mischungen untereinander oder in Kombination mit geeigneten Hilfs- und/oder Trägerstoffen verabreicht werden.

Gegenstand der Erfindung sind somit auch Arzneimittel, die aus mindestens einer Verbindung der Formel I, gegebenenfalls in Form eines ihrer Säureadditionssalze,bestehen oder mindestens eine dieser Wirkstoffe neben pharmazeutisch geeigneten und physiologisch verträglichen Trägerstoffen, Verdünnungsmitteln und/oder anderen Hilfsstoffen enthalten.

Die erfindungsgemäßen Arzneimittel können oral, topisch, rektal oder gegebenenfalls auch parenteral appliziert werden, wobei die orale Anwendung bevorzugt ist.
Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyäthylenglykole und Lösungsmittel, wie etwa steriles Wasser und ein- oder mehrwertige Alkohole, z. B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formel I und/oder mindestens eines von deren physiologisch verträglichen Säureadditionssalzen enthält. Bei festen Dosierungseinheiten, wie Tabletten, Kapseln, Dragees oder Suppositorien, kann diese Dosis bis zu etwa 800 mg, bevorzugt jedoch etwa 100 bis 500 mg betragen.

Für die Behandlung eines an entzüdlich rheumatischen Erkrankungen leidenden, erwachsenen Patienten sind - je nach Wirksamkeit der Verbindungen gemäß Formel I und/oder der entsrechenden Säureadditionssalze am Menschen - Tagesdosen von etwa 100 bis 2000 mg Wirkstoff, vorzugsweise etwa 300 bis 1000 mg, bei oraler Verabreichung indiziert. Unter Umständen können jedoch auch höhere oder niedrigere Tagesdosen angebracht sein. Die Verabreichung der Tagesdosis kann sowohl durch Einmalgabe in Form einer einzlenen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I und deren entsprechende Säureadditionssalze bei der Herstellung der vorgenannten galenischen Zubereitungsformen auch zusammen mit anderen geeigneten Wirkstoffen, beispielsweise Antiuricopathika, Thrombocytenaggregationshemmern, Analgetika und anderen steroidalen oder nicht-steroidalen Antiphlogistika, formuliert werden.

Die Struktur aller nachstehend beschriebenen Verbindungen wurde durch Elementaranalyse und IR- sowie $^1$H-NMR-Spektren gesichert.

Beispiel 1: 3-(3,5-Di-tert.butyl-4-hydroxyphenyl)-6,7-dihydro-2H-imidazo[2,1-b][1,3,4]thiadiazinhydrochlorid

a) 2-Brom-1-(3,5-di-tert.butyl-4-hydroxyphenyl)-äthanon

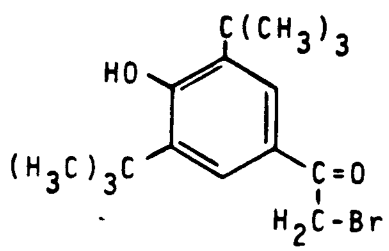

206 g (0,83 mol) 1-(3,5-Di-tert.butyl-4-hydroxyphenyl)-äthanon wurden unter Rühren in 415 ml Methylenchlorid gelöst, zum Sieden erhitzt und innerhalb von 30 Minuten tropfenweise mit 144 g (0,9 mol) Brom versetzt. Danach wurde für weitere 2 Stunden unter Rückfluß erhitzt, die Mischung abgekühlt, mit 400 ml Wasser versetzt, die organische Phase abgetrennt und über Natriumsulfat getrocknet. Nach dem Entfernen des Lösungsmittels unter vermindertem Druck wurde das erhaltene feste Rohprodukt aus 540 ml Methylcyclohexan umkristallisiert.

Ausbeute: 191 g (67 % der Theorie)
Schmelzpunkt: 105 - 108° C
$C_{16}H_{23}BrO_2$ (MG = 327,3)

b) 3-(3,5-Di-tert.butyl-4-hydroxyphenyl)-6,7-dihydro-2H-imidazo [2,1 -b][1,3,4]thiadiazin-hydrochlorid

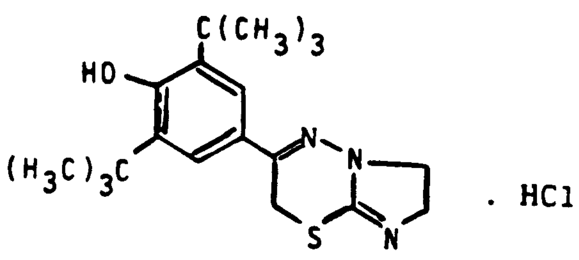

65,5 g (0,2 mol) 2-Brom-1-(3,5-di-tert.butyl-4-hydroxyphenyl)-äthanon aus Stufe a) und 23,4 g (0,2 mol) 1-Amino-2-thioxoimidazolidin wurden in 600 ml Äthanol gelöst und 2 Stunden zum Sieden erhitzt. Der dabei entstandene Niederschlag wurde abgesaugt, in 500 ml Wasser aufgenommen und mit 2 N Natronlauge alkalisch gestellt. Extraktion mit Dichlormethan, Trocknen der organischen Phase über Natriumsulfat und Einengen unter reduziertem Druck ergab 57,1 g freie Base. Das Produkt wurde in 250 ml Chloroform gelöst und mit ätherischer Salzsäure in das Hydrochlorid übergeführt, das als kristalliner Niederschlag abfiltriert

und im Vakuum getrocknet wurde.

Ausbeute: 73,6 g (95,5 % der Theorie)
Schmelzpunkt: 262 - 264° C (Zers.)
$C_{19}H_{28}ClN_3OS$ (MG = 382,0)

Analyse: Berechnet: C 59,75 %; H 7,39 %; Cl 9,28 %; N 11,00 %; S 8,39 %
Gefunden: C 59,77 %; H 7,62 %; Cl 9,55 %; N 11,03 %; S 8,43 %

Beispiel 2: 3-(3,5-Di-tert.butyl-4-hydroxyphenyl)-2-methyl-6,7-dihydro-2H-imidazo[2,1-b][1,3,4]-thiadiazinhydrochlorid

a) 2-Brom-1-(3,5-di-tert.butyl-4-hydroxyphenyl)-propanon

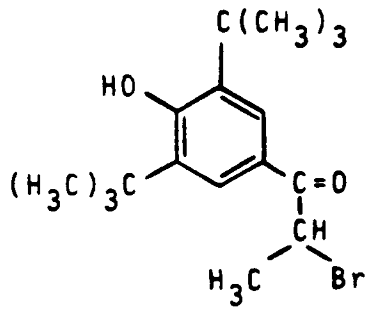

Zu einer siedenden Suspension von 139,0 g (0,62 mol) Kupfer-II-bromid in 300 ml Essigsäureäthylester tropfte man unter Rühren eine Lösung von 82,0 g (0,31 mol) 1-(3,5-Di-tert.butyl-4-hydroxyphenyl)-propanon in 300 ml Chloroform. Anschließend wurde bis zur Beendigung der Bromwasserstoff-Entwicklung 3 Stunden unter Rückfluß erhitzt. Nach Abkühlen auf Raumtemperatur wurden die Kupfersalze abgesaugt, der Filterrückstand zweimal mit Essigsäureäthylester nachgewaschen und das Filtrat unter vermindertem Druck eingeengt. Die Umkristallisation des festen Rückstandes erfolgte aus Petroläther (40 - 60° C).

Ausbeute: 87,5 g (82 % der Theorie)
Schmelzpunkt: 130 - 132° C
$C_{17}H_{25}BrO_2$ (MG = 341,3)

b) 3-(3,5-Di-tert.butyl-4-hydroxyphenyl)-2-methyl-6,7-dihydro-2H-imidazo[2,1-b][1,3,4]thiadiazin-hydrochlorid

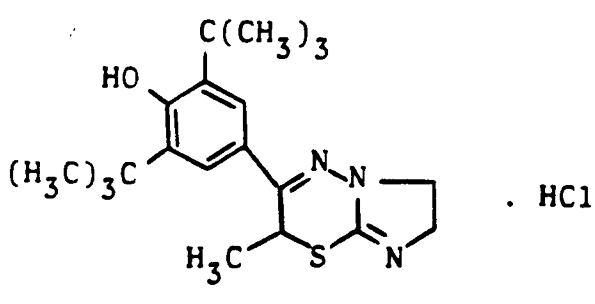

17,1 g (0,05 mol) 2-Brom-1-(3,5-di-tert.butyl-4-hydroxyphenyl)-propanon aus Stufe a) und 5,5 g (0,047 mol) 1-Amino-2-thioxoimidazolidin wurden in 150 ml Äthanol 5 Stunden unter Rückfluß erhitzt. Nach Abkühlen wurde die Lösung eingedampft, mit 200 ml Wasser versetzt und mit 2 N Natronlauge alkalisch gestellt. Extraktion mit Dichlormethan, Trocknen und Einengen ergab ölige Rohbase, die in 100 ml Essigsäureäthylester gelöst und nach Zugabe einer äquimolaren Menge an ätherischer Salzsäure als Hydrochlorid isoliert wurde.

Ausbeute: 144,4 g (77,4 % der Theorie)
Schmelzpunkt: 247 - 249° C
$C_{20}H_{30}ClN_3OS$ (MG = 396,0)

Analyse: Berechnet: C 60,66 %; H 7,64 %; Cl 8,95 %; N 10,61 %; S 8,10 %
Gefunden: C 60,44 %; H 7,66 %; Cl 9,28 %; N 10,56 %; S 7,98 %

Beispiel 3: 3-(5-tert.Butyl-3-methyl-4-hydroxyphenyl)-6,7-dihydro-2H-imidazo[2,1-b][1,3,4]-thiadiazinhydrochlorid

a ) 2-Brom-1-(5-tert.butyl-3-methyl-4-hydroxyphenyl)-äthanon

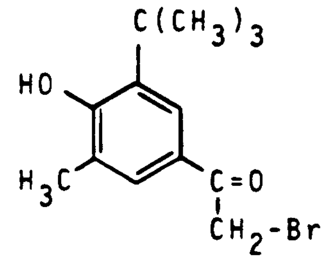

Eine zum Sieden erhitzte Suspension von 179 g (0,8 mol) Kupfer(II)bromid in 360 ml Essigsäureäthylester wurde unter Rühren tropfenweise mit einer Lösung von 82,5 g (0,4 mol) 1-(5-tert.Butyl-3-methyl-4-hydroxyphenyl)-äthanon in 360 ml Chloroform versetzt. Anschließend wurde bis zur Beendigung der Bromwasserstoff-Entwicklung 4 Stunden lang unter Rückfluß erhitzt. Nach dem Abkühlen auf Raumtemperatur wurden die Kupfersalze abgesaugt, der Filterrückstand mehrmals mit Essigsäureäthylester gewaschen, das Filtrat unter vermindertem Druck eingeengt und der feste Rückstand aus Cyclohexan umkristallisiert.

Ausbeute: 81,9 g (72 % der Theorie)
Schmelzpunkt: 90 - 92 ° C
$C_{13}H_{17}BrO_2$ (MG = 285,2)

b) 3-(5-tert.Butyl-3-methyl-4-hydroxyphenyl)-6,7-dihydro-2H-imidazo[2,1-b][1,3,4]thiadiazin-hydrochlorid

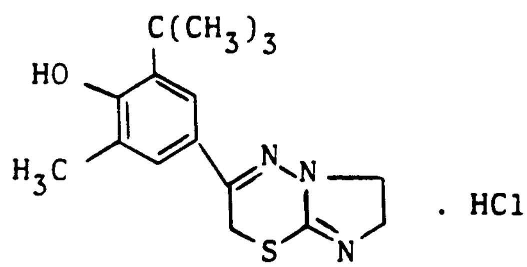

18,3 g (0,064 mol) 2-Brom-1-(5-tert.butyl-3-methyl-4-hydroxyphenyl)-äthanon aus Stufe a) und 7,0 g (0,06 mol) 1-Amino-2-thioxoimidazolidin wurden in 180 ml Äthanol gelöst und 2 Stunden unter Rückfluß erhitzt. Nach Einengen der Reaktionsmischung zur Trockene wurde der Rückstand in 200 ml Wasser aufgenommen, mit 2 N Natronlauge neutralisiert und mit Chloroform dreimal ausgeschüttelt. Man dampfte die vereinigten organischen Extrakte ein, löste den Rückstand in 200 ml siedendem Äthanol und versetzte mit äthanolischer Salzsäure. Das dabei kristallin angefallene Hydrochlorid wurde abfiltriert und aus Isopropanol umkristallisiert.

Ausbeute: 15,7 g (77 % der Theorie)
Schmelzpunkt: 234° C (Zers.)
$C_{16}H_{22}ClN_3OS$ (MG = 339,9)

Analyse: Berechnet: C 56,55 %; H 6,53 %; Cl 10,43 %; N 12,36 %; S 9,43 %
Gefunden: C 56,23 %; H 6,46 %; Cl 10,16 %; N 12,62 %; S 9,39 %

Beispiel 4: 3-(3,5-Di-tert.butyl-4-hydroxyphenyl)-2H-imidazo[2,1-b][1,3,4]thiadiazin-hydrochlorid

a) 1-Amino-2-mercapto-imidazol-hydrochlorid

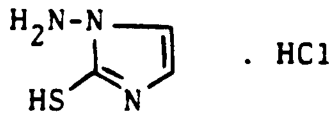

Zu einer Lösung von 29 ml (0,9 mol) wasserfreiem Hydrazin in 180 ml Äthanol tropfte man unter Kühlung im Eisbad eine Lösung von 134 g (0,9 mol) Thioisocyanato-acetaldehyde-dimethylacetal so hinzu, daß die Reaktionstemperatur 30° C nicht überschritt. Nach 2-stündigem Erhitzen unter Rückfluß und anschließendem Abkühlen auf Raumtemperatur wurden 113 ml konz. Salzsäure zugetropft und weitere 2 Stunden zum Sieden erhitzt. Der dabei gebildete Niederschlag wurde abgesaugt und aus Isopropanol umkristallisiert.

Ausbeute: 123,7 g (91 % der Theorie)
Schmelzpunkt: 167 - 168° C
$C_3H_6ClN_3S$ (MG = 151,6)

b) 3-(3,5-Di-tert.butyl-4-hydroxyphenyl)-2H-imidazo[2,1-b][1,3,4]thiadiazin-hydrochlorid

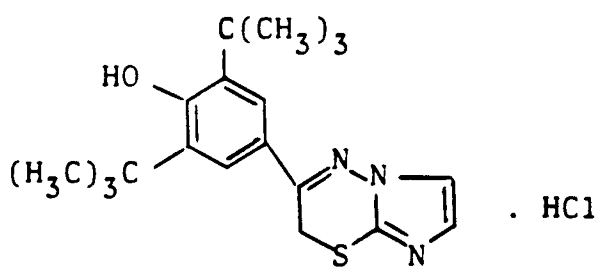

Eine Lösung von 18,0 g (0,055 mol) 2-Brom-1-(3,5-di-tert.butyl-4-hydroxyphenyl)-äthanon und 7,6 g (0,05 mol) 1-Amino-2-mercapto-imidazol-hydrochlorid aus Stufe a) in 150 ml Äthanol wurden 9 Stunden unter Rückfluß erhitzt. Der beim Eindampfen der Lösung verbleibende Rückstand wurde mit 50 ml 2 N Natronlauge alkalisch gestellt und mit Chloroform extrahiert. Zur Überführung in das Hydrochlorid versetzte man die getrocknete Chloroformphase mit einer äquimolaren Menge an ätherischer Salzsäure, saugte den angefallenen Kristallbrei ab und kristallisierte aus Äthanol um.

Ausbeute: 12,0 g (63 % der Theorie)
Schmelzpunkt: 219 - 220° C
$C_{19}H_{26}ClN_3OS$ (MG = 380,0)

Analyse: Berechnet:C 60,06 %; H 6,90 %; Cl 9,33 %; N 11,06 %; S 8,44 %
Gefunden: C 60,09 %; H 6,98 %; Cl 9,47 %; N 11,01 %; S 8,51 %

Analog Beispiel 4 wurde das 3-(3,5-Di-tert.butyl-4-hydroxyphenyl)-2-methyl-2H-imidazo[2,1-b][1,3,4]-thiadiazin-hydrochlorid vom Schmelzpunkt 202 - 205° C aus 2-Brom-1-(3,5-di-tert.butyl-4-hydroxyphenyl)-propanon des Beispiels 2 a) und 1-Amino-2-mercapto-imidazol dargestellt.

Beispiel 5: 3-(3,5-Di.tert.butyl-4-hydroxyphenyl)-2-methyl-2H-s-triazolo[3,4-b][1,3,4]thiadiazin

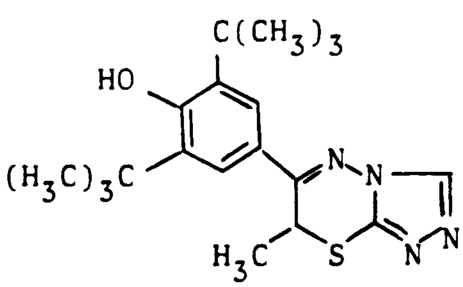

19,0 g (0,046 mol) 2-Brom-1-(3,5-di.tert.butyl-4-hydroxyphenyl)-propanon aus Beispiel 2 a) und 5,8 g (0,05 mol) 4-Amino-3-mercapto-1,2,4-triazol wurden in 120 ml Äthanol 5 Stunden unter Rückfluß erhitzt und nach dem Abkühlen zur Trockene eingedampft. Der ölige Rückstand verfestigte sich nach Zugabe von 100 ml Essigsäureäthylester und bei intensivem Rühren zu einem Kristallbrei, der dann aus Isopropanol umkristallisiert wurde.

| | |
|---|---|
| Ausbeute: | 12,9 g (72 % der Theorie) |
| | Schmelzpunkt: 195° C |
| | $C_{19}H_{26}N_4OS$ (MG = 358,5) |
| Analyse: | Berechnet: C 63,66 %; H 7,31 %; N 15,63 %; S 8,94 % |
| | Gefunden: C 63,90 %; H 7,48 %; N 15,61 %; S 9,02 % |

Entsprechend Beispiel 5 erfolgte die Herstellung des 3-(3,5-Di-tert.butyl-4-hydroxyphenyl)-2H-s-triazolo-[3,4-b][1,3,4]thiadiazins vom Schmelzpunkt 263° C aus 2-Brom-1-(3,5-di-tert.butyl-4-hydroxyphenyl)-äthanon des Beispiels 1 a) und 4-Amino-3-mercapto-1,2,4-triazol.

Beispiel 6: 3-(3,5-Di-tert.butyl-4-hydroxyphenyl)-7-oxo-6,7-dihydro-2H-imidazo[2,1-b][1,3,4]-thiadiazinhydrobromid

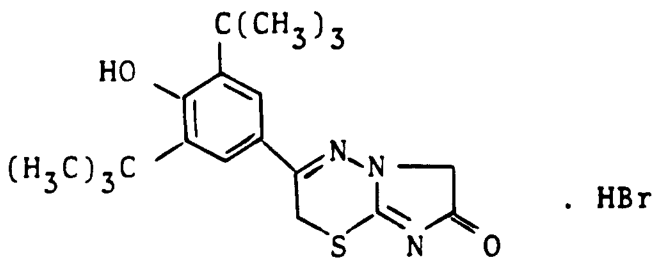

36,0 g (0,11 mol) 2-Brom-1-(3,5-di-tert.butyl-4-hydroxyphenyl)-äthanon aus Beispiel 1a) und 16,7 g (0,1 mol) 1-Amino-2-thioxo-4-imidazolidinon wurden in 300 ml Eisessig unter Rühren auf 90° C erwärmt. Nachdem im Laufe von etwa 20 Minuten eine klare Lösung entstanden war, ließ man auf Raumtemperatur abkühlen. Das hierbei angefallene Kristallisat wurde abgesaugt und aus Eisessig umkristallisiert.

| | |
|---|---|
| Ausbeute: | 29,5 g (67 % der Theorie) |
| | Schmelzpunkt: 208 - 209° C (Zers.) |
| | $C_{19}H_{26}BrN_3O_2S$ (MG = 440,4) |
| Analyse: | Berechnet: C 51,82 %; H 5,95 %; Br 18,14%; N 9,54 %, S 8,28 % |
| | Gefunden: C 51,89 %; H 5,88 %; Br 17,85 %; N 9,46 %; S 7,43 % |

Beispiel 7: 3-(3,5-Di-tert.butyl-4-hydroxyphenyl-6-oxo-6,7-dihydro-2H-imidazo[2,1-b][1,3,4]thiadiazin-hydrobromid

a) 1-Amino-2-thioxo-5-imidazolidinon

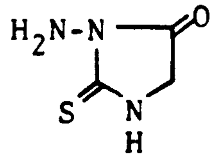

21,6 g (0,12 mol) Thiosemicarbazido-(4)-essigsäureäthylester in 285 ml Äthanol wurden nach Zugabe von 7,2 ml 1 N Natriumäthanolat-Lösung 15 Minuten unter Rückfluß erhitzt. Nach Versetzen der noch heißen Lösung mit 7 ml 1 N Salzsäure und weiteren 660 ml Äthanol wurde bis zur vollständigen Lösung des entstandenen Niederschlages kurzzeitig erhitzt. Nach 6 Stunden Rühren bei Raumtemperatur wurden die ausgeschiedenen Gelblichen Kristalle abfiltriert, mit Äthanol und Diäthyläther gewaschen und im Vakuum getrocknet.

| | |
|---|---|
| Ausbeute: | 13,8 g (88 % der Theorie) |

Schmelzpunkt: 168 - 169° C
$C_3H_5N_3OS$ (MG = 131,2)

b) 3-(3,5-Di-tert.butyl-4-hydroxyphenyl)-6-oxo-6,7-dihydro-2H-imidazo[2,1-b][1,3,4]thiadiazin-hydrobromid

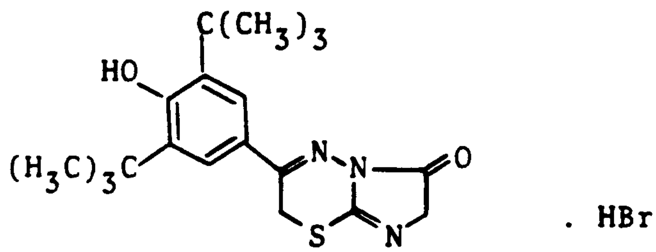

34,4 g (0,11 mol) 2-Brom-1-(3,5-di-tert.butyl-4-hydroxyphenyl)-äthanon aus Beispiel 1a) und 13,8 g (0,11 mol) 1-Amino-2-thioxo-5-imidazolidinon aus Stufe a) wurden in 300 ml Eisessig 30 Minuten auf 90° C erhitzt. Nach dem Erkalten filtrierte man den Niederschlag ab, wusch mit 250 ml Essigsäureäthylester nach und trocknete die Kristalle im Vakuum über Phosphorpentoxid.

Ausbeute: 30,4 g (63 % der Theorie)
Schmelzpunkt: 235 - 236° C (Zers.)
$C_{19}H_{26}BrN_3O_2S$ (MG = 440,4)
Analyse: Berechnet: C 51,82 %; H 5,95 %; Br 18,14 %; N 9,54 % S 7,28%
Gefunden: C 51,51 %; H 5,74 %; Br 17,74 %; N 9,28 %; S 7,24 %

Pharmakologische Prüfung und Ergebnisse

Die Prüfung der erfindungsgemäßen Verbindungen der Formel I auf antiphlogistische Wirkung, Beeinflussung immunpathologischer Prozesse, Sauerstoffradikale desaktivierende Potenz, ulzerogene Aktivität und akute Toxizität erfolgte in den anschließend beschriebenen Tiermodellen, wobei das in der Rheumatherapie zu den Standardpräparaten der ersten Wahl zählende Antiphlogistikum Naproxen (2-(6-Methoxy-2-naphthyl)-propionsäure) als Vergleichssubstanz in die Untersuchungen miteinbezogen wurde.

1. Adjuvans-Arthritis

Die Untersuchungen wurde nach der Methode von Pearson (Arthrit. Rheum. 2 1959),S. 44) durchgeführt. Als Versuchstiere dienten männliche Ratten eines Wistar-Lewis-Stammes mit einem Körpergewicht zwischen 130 und 200 g. Die zu prüfenden Verbindungen wurden in Dosen von 50 mg pro kg Körpergewicht einmal täglich vom 1. bis zum 5. Versuchstag oral (p. o.) appliziert. Die Tiere einer Kontrollgruppe erhielten nur das Vehikel. Jede Präparat- und die Kontrollgruppe umfaßte 8 Tiere. Als Wirkungskriterium diente die prozentuale herabsetzung der Pfotenvolumenzunahme gegenüber jener der unbehandelten Kontrollgruppe.

2. Akute gastrale Ulzerogenität

Die Untersuchung erfolgte an jeweils 10 männlichen Sprague-Dawley Ratten mit durch Hungerstreß sensibilisierter Magenschleimhaut. Das Körpergewicht der Tiere lag zwischen 200 und 300 g. 48 Stunden vor Applikation der Prüfpräparate bis zum Töten der Tiere wurde bei freiem Zugang zum Trinkwasser das Futter entzogen. Die Ratten wurden 24 Stunden nach oraler Substanzgabe getötet, die Mägen entnommen, unter fließendem Wasser gereinigt und auf Schleimhautläsionen inspiziert.

Als Ulzera galten alle makroskopisch sichtbaren Läsionen. Bestimmt wurde die Anzahl der Tiere mit Ulzera je Dosis und daraus die $UD_{50}$-Werte, also jene Dosen, mit denen bei 50 % der Tiere Läsionen hervorgerufen wurden, nach Litchfield und Wilcoxon (J. Pharmacol. exp. Ther. 96 (1949),S. 99) berechnet.

3. Akute Toxizität

Die Bestimmung der $LD_{50}$-Werte erfolgte standardgemäß über die innerhalb von 7 Tagen bei NMRI-

(Naval Medical Research Institute)-Mäusen (6 Tiere pro dosi) nach einmaliger intraperitonealer (i. p.) Gabe auftretende Mortalität.

Die Ergebnisse dieser Untersuchungen, die die Überlegenheit der erfindungsgemäßen Verbindungen der Formel I gegenüber dem Standardpräparat Naproxen eindeutig belegen, sind in der nachstehenden Tabelle 1 zusammengefaßt.

Tabelle 1: Antiphlogistische Wirkung, Ulzerogenität und akute Toxizität

| Verbindung aus Beispiel | Adjuvans-Arthritis (% Hemmung bei 50 mg/kg p.o.) | Akute Ulzerogenität $UD_{50}$ (mg/kg) | Akute Toxizität $LD_{50}$ (mg/kg) |
|---|---|---|---|
| 1 | 62 | 400 | > 1200 |
| 2 | 63 | 200 | > 1200 |
| 4 | 69 | > 200 *) | 300 - 600 |
| 5 | 56 | > 200 *) | > 1200 |
| 6 | 73 | > 200 *) | 300 - 600 |
| 7 | 59 | > 200 *) | 300 - 600 |
| Naproxen | 55 | 23 | 500 |

*) jeweils verabreichte Höchstdosis

Besonders augenfällig wird die Überlegenheit der erfindungsgemäßem Verbindungen beim Vergleich der gastralen Verträglichkeit, der bei der Behandlung von entzündlich rheumatischen Erkrankungen mit nicht-steroidalen Antiphlogistika eine entscheidende klinische Bedeutung zukommt.

Auch in weiteren speziellen Versuchen erwiesen sich die erfindungsgemäßen Verbindungen dem standardpräparat Naproxen eindeutig überlegen.

4. Hemmung immunpathologischer Prozesse

Es ist heute allgemein anerkannt, daß der progrediente Verlauf der entzündlich rheumatischen Erkrankungen hauptsächlich durch Entgleisungen im Immunsystem verursacht wird und daß eine kausale Therapie nur mit solchen Medikamenten gelingen kann, die diese immunpathologischen Prozesse zu durchbrechen vermögen.

a) Adjuvans Arthritis

In dem unter Punkt 1 beschriebenen Rattenmodell der mit Freundschem Adjuvans induzierten Arthritis ist gewöhnlich die Immunaktivität der Lymphocyten gegenüber bestimmten Mitogenen, wie Concavalin-A, Phytohämagglutinin-A und Dextransulfat, drastisch herabgesetzt. Es wurde daher die stimulierende Wirkung auf diese stark supprimierte Immunantwort untersucht. Hierbei bewirkte beispielsweise die Verbindung aus Beispiel 1 nach oraler Verabreichung von 20 mg/kg eine weitgehende Normalisierung der Immunreaktivität, während das in Dosen bis zu 25 mg/kg geprüfte Naproxen wirkungslos war.

b) Typ II-Collagen-induzierte Arthritis

In diesem Versuche wurde die Arthritis an männlichen Wistar Ratten mit Collagen vom Typ II ausgelöst, das sich nach Standardmethoden von Miller und Rhodes (Meth. Enzymol. 82 (1982),S. 33) aus der Nasenscheidewand des Kalbes gewinnen ließ und den Tieren im Gemisch mit inkomplettem Freundschem Adjuvans intradermal injiziert wurde. Dieser Immunisierungsvorgang wurde 7 Tage später wiederholt. 20 Tage nach der Erstimmunisierung teilte man die erkrankten Ratten in Gruppen zu jeweils 7 Tieren auf, die in der anschließenden 20-tägigen Behandlungsphase einmal täglich die jeweilige Prüfsubstanz bzw. das reine Vehikel (Kontrollgruppe) oral erhielten.

Am 41. Versuchstag, also einen Tag nach der letzten Substanzgabe, wurden aus der Milz der Versuchstiere Lymphocyten gewonnen und deren Immunaktivität gegenüber Mitogenen untersucht, da auch in diesem Modell der Immunstatus der Lymphocyten empfindlich gestört ist.
Für die erfindungsgemäßen Verbindungen ließen sich wiederum dosisabhängige kurative Effekte auf das stark geschwächte Immunsystem nachweisen, während Naproxen keine Wirkung entfaltete. So wurde beispielsweise mit einer Dosis von 25 mg/kg p. o. der Verbindung aus Beispiel 1 die Immunfunktion sowohl der T- als auch der B-Lymphocyten vollständig normalisiert.

5. Antioxydative Wirkung

Nach heutiger Lehrmeinung sind an dem multifaktoriell bedingten, progressiven Verlauf der rheumatoiden Arthritis und anderer entzündlicher Erkrankungen aggressive Sauerstoffradikale maßgeblich beteiligt, die während des chronischen Entzündungsprozesses exzessiv gebildet werden und selbst als hochtoxische Entzündungsmediatoren die über eine irreversible Lipidperoxydation der Zellmembranen verlaufende Bindegewebszerstörung perpetuell unterhalten. Folglich sollten antioxydativ wirksame Pharmaka mit der Fähigkeit zur Desaktivierung dieser extrem cytotoxischen Sauerstoffradikale einen gezielten Eingriff in das chronische Entzündungsgeschehen gestatten. Ein geeignetes Tiermodell für eine derartige durch Sauerstoffradikale vermittelte Gewebszerstörung stellt die an der Ratte mit Adriamycin (Doxorubicin) induzierte Entzündung dar.

a) Adriamycin-induzierte Entzündung

Die Untersuchungen wurden nach der Methode von D.M. Siegel et al. (Inflammation 4 (1980),S. 233) an männlichen Sprague-Dawley Ratten mit einem Körpergewicht zwischen 200 und 230 g in Gruppen von jeweils 7 Tieren durchgeführt, die 0,1 mg Adriamycin, gelöst in 0,1 ml 0,9%iger Kochsalzlösung, durch subkutane Injektion in die linke Hinterpfote erhielten. 72 Stunden danach wurde die Pfotenvolumenzunahme als Maß für den Entzündungsgrad durch plethysmographische Messung bestimmt.
Die orale Verabreichung der Prüfpräparate in 1%iger wäßriger Carboxymethylcellulose-Suspension erfolgte einmal täglich an 4 aufeinanderfolgenden Tagen, beginnend mit dem Tag der Adriamycin-Injektion.

Auch in diesem Test zeigte beispielsweise die Verbindung aus Beispiel 1 einen dosisabhängigen Schutzeffekt gegen die durch Adriamycin ausgelöste Gewebszerstörung, der bei einer Dosis von 80 mg/kg p. o. einen Hemmwert von 55 % erreichte. Sowohl die bekannten steroidalen als auch nicht-steroidalen Antiphlogistika, einschließlich Naproxen, sind in dieser Versuchsanordnung unwirksam.

b) In-vitro-Hemmung der Lipidperoxydation

Einen weiteren überzeugenden Beleg für die ausgeprägte Schutzwirkung der erfindungsgemäßen Verbindungen gegenüber den aggressiven Sauerstoffradikalen lieferte der Thiobarbitursäure-Test nach A. Ottolenghi (Arch. Biochem. Biophys. 79 (1959) S. 355-363). Mit dieser In-vitro-Methode läßt sich anhand des beim oxydativen Abbau membrangebundener, mehrfach ungesättigter Fettsäuren entstehenden Malondialdehyds die Beeinflussung sowohl der mikrosomalen als auch der mitochondrialen Lipidperoxydation durch antioxydativ wirksame Präparate bestimmen.

Auch hier übten die Verbindungen der Formel I eine starke Hemmwirkung aus. Für die Verbindung des Beispiels 1 ergaben sich mit Mikrosomen und Mitochondrien aus der Rattenleber beispielsweise $IC_{50}$-Werte von $5 \bullet 10^{-7}$ bzw. $4 \bullet 10^{-6}$ mol/l.

6. Hemmung der 5-Lipoxygenase

Die Hemmwirkung der erfindungsgemäßen Verbindungen auf den durch die 5-Lipoxygenase katalysierten Arachidonsäure-Abbau wurde, wie üblich, in In-vitro-Versuchen an isolierten polymorphkernigen Humangranulozyten nachgewiesen. Hierzu wurden die durch den Calcium-Ionophor A 23 187 (Calbiochem GmbH, Frankfurt/Main, BRD, Biochemical and Immunochemical Catalog 1985, S. 284) stimulierten Zellen mit $^{14}C$-markierter Arachidonsäure inkubiert und die nach 15 Minuten bei 37 ° C durch Biotransformation gebildeten radioaktiven Hauptabbauprodukte der Arachidonsäure, die 5-Hydroxyeicosatetraensäure (5-HETE) und das besonders stark proinflammatorisch wirkende Leukotrien $B_4$ ($LTB_4$), nach Auftrennung durch Hochdruckflüssigkeitschromatographie (HPLC) mit Hilfe eines Radio-Monitors quantitativ bestimmt.
In dieser Versuchsanordnung ließ sich die Bildung sowohl von $LTB_4$ als auch 5-HETE und demzufolge der Arachidonsäure-Abbau über die 5-Lipoxygenase durch 15-minütige Vorinkubation der Granulozyten etwa mit der Verbindung des Beispiels 1 im Konzentrationsbereich zwischen $10^{-5}$ und $10^{-6}$ mol/l signifikant hemmen.

**Patentansprüche**
**Patentansprüche für folgende Vertragsstaaten : AT, BE, CH, DE, FR, GB, IT, LI, LU, NL, SE**

**1.** Imidazo- und Triazolo-thiadiazine der allgemeinen Formel I

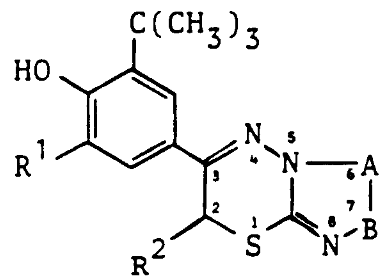

(I),

in der

$R^1$ eine geradkettige oder verzweigte Alkylgruppe mit 1 bis 4 C-Atomen und
$R^2$ ein Wasserstoffatom oder einen Alkylrest mit 1 bis 3 C-Atomen bedeuten und das zweigliedrige Strukturelement
-A-B- im Fünfring für $-CH_2-CH_2-$, -CH = CH-, -CH = N-, $-CH_2-CO-$oder $-CO-CH_2-$ steht,

sowie deren physiologisch verträglichen Säureadditionssalze.

**2.** Verbindungen nach Anspruch 1, gekennzeichnet durch wenigstens eines der Merkmale, daß das Strukturelement -A-B- eine Äthylen-oder Vinylengruppe bedeutet oder aber entweder A oder B für eine Carbonylgruppe und das andere Brückenglied für eine Methylengruppe stehen.

**3.** Verbindungen nach Anspruch 1 oder 2, dadurch gekennzeichnet, daß $R^1$ einen tert.Butylrest bedeutet und $R^2$ für Wasserstoff oder Methyl steht.

**4.** Verbindung nach Anspruch 3, dadurch gekennzeichnet, daß sie 3-(3,5-Di-tert.butyl-4-hydroxyphenyl)-6,7-dihydro-2H-imidazo [2,1-b][1,3,4]thiadiazin darstellt.

**5.** Verfahren zur Herstellung der Imidazo- und Triazolothiadiazine der Formel I und ihrer physiologisch verträglichen Säureadditionssalze gemäß einem oder mehreren der Ansprüche 1 bis 4, dadurch gekennzeichnet, daß man ein 2-Halogen-1-phenylalkanon der Formel II

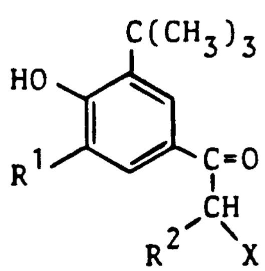

(II)

mit einer Verbindung der Formel III,

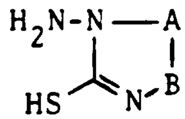

(III)

wobei $R^1$, $R^2$ und das zweigliedrige Strukturelement -A-B- die im Anspruch 1 genannten Bedeutungen haben und X für ein Halogenatom steht, zu den Verbindungen der Formel I umsetzt und diese entweder in freier Form isoliert oder mit geeigneten Säuren in physiologisch verträgliche Additionssalze umwandelt.

**6.** Verbindungen der Formel I sowie deren physiologisch verträgliche Säureadditionssalze gemäß einem oder mehreren der Ansprüche 1 - 4 zur Anwendung als Heilmittel.

**7.** Arzneimittel, gekennzeichnet durch einen Gehalt an - oder bestehend aus - mindestens einer Verbindung der Formel I und/oder einem ihrer physiologisch verträglichen Säureadditionssalze nach einem oder mehreren der Ansprüche 1 bis 4.

**8.** Arzneimittel nach Anspruch 7, dadurch gekennzeichnet, daß es zur Vorbeugung und Behandlung von Krankheiten bestimmt ist, bei denen die therapeutische Anwendung von Entzündungshemmern, Immunmodulatoren, Sauerstoffradikale desaktivierenden Mitteln und/oder Hemmstoffen des durch die 5-Lipoxygenase vermittelten Arachidonsäureabbaus indiziert ist.

**9.** Arzneimittel nach Anspruch 7 oder 8, dadurch gekennzeichnet, daß es zur Voprbeugung und Behandlung von entzündlichen, insbesondere von entzündlich rheumatischen Erkrankungen verwendet wird.

**10.** Verfahren zur Herstellung eines Arzneimittels gemäß einem oder mehreren der Ansprüche 7 - 9, dadurch gekennzeichnet, daß man mindestens eine Verbindung der Formel I und/oder mindestens ein physiologisch verträgliches Säureadditionssalz einer solchen Verbindung
mit einem physiologisch annehmbaren Träger und gegebenenfalls weiteren Zusatz- und/oder Hilfsstoffen in eine geeignete Darreichungsform bringt.

**11.** Verwendung von Verbindungen der Formel I und/oder von deren physiologisch verträglichen Säureadditionssalzen zur Herstellung von Arzneimitteln gemäß den Ansprüchen 8 und 9.

**12.** 1-Amino-2-mercapto-imidazol und dessen tautomere Form der Formeln

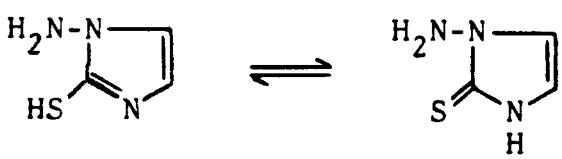

**13.** Verfahren zur Herstellung des 1-Amino-2-mercapto-imidazols gemäß Anspruch 12, dadurch gekennzeichnet, daß man ein Thioisocyanatoacetaldehyddialkylacetal mit Hydrazin umsetzt.

**14.** 1-Amino-2-thioxo-5-imidazolidinon und dessen tautomere Formen der Formeln

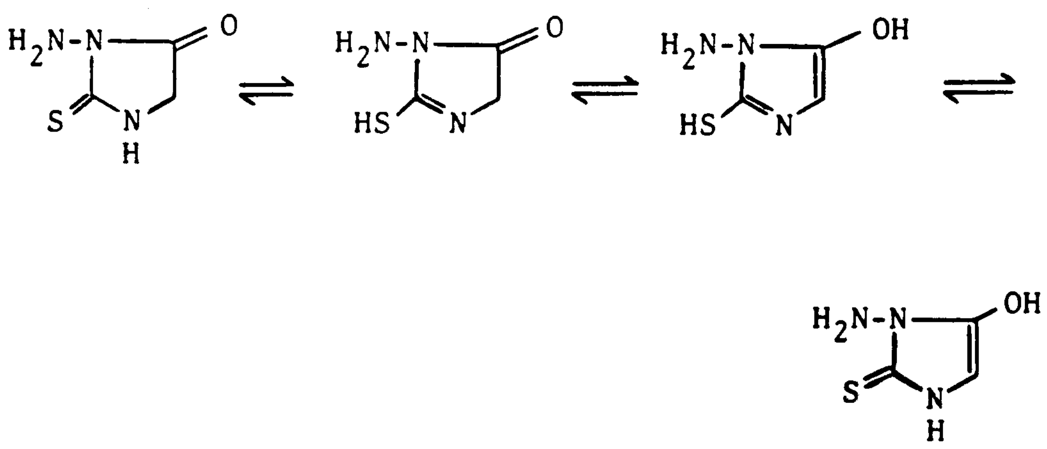

**15.** Verfahren zur Herstellung des 1-Amino-2-thioxo-5-imidazolidinons gemäß Anspruch 14, dadurch gekennzeichnet, daß man einen Thiosemicarbazido-(4)-essigsäurealkylester alkalisch cyclisiert.

**Patentansprüche für folgende Vertragsstaaten : ES, GR**

**1.** Verfahren zur Herstellung von Imidazo- und Triazolothiadiazinen der allgemeinen Formel I

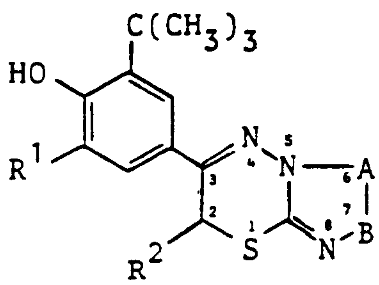

(I),

in der

- $R^1$ eine geradkettige oder verweigte Alkylgruppe mit 1 bis 4 C-Atomen und
- $R^2$ ein Wasserstoffatom oder einen Alkylrest mit 1 bis 3 C-Atomen bedeuten und das zweigliedrige Strukturelement
- -A-B- im Fünfring für $-CH_2-CH_2-$, -CH = CH-, -CH = N-, $-CH_2-CO-$oder $-CO-CH_2-$ steht,

sowie von deren physiologisch verträglichen Säureadditionssalzen, dadurch gekennzeichnet, daß man ein 2-Halogen-1-phenylalkanon der Formel II

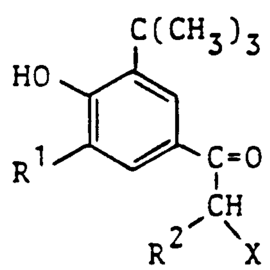

(II)

mit einer Verbindung der Formel III,

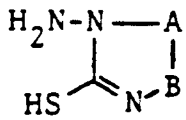

(III)

wobei $R^1$, $R^2$ und das zweigliedrige Strukturelement -A-B- die bei Formel I genannten Bedeutungen haben und X für ein Halogenatom steht, zu den Verbindungen der Formel I umsetzt und diese entweder in freier Form isoliert oder mit geeigneten Säuren in physiologisch verträgliche Additionssalze unwandelt.

**2.** Verfahren nach Anspruch 1, dadurch gekennzeichnet, daß man Verbindungen der Formel I mit wenigstens einem der Merkmale, daß das Strukturelement -A-B- eine Äthylen- oder Vinylengruppe bedeutet oder aber entweder A oder B für eine Carbonylgruppe und das andere Brückenglied für eine Methylengruppe stehen, herstellt.

**3.** Verfahren nach Anspruch 1 oder 2, dadurch gekennzeichnet, daß man Verbindungen der Formel I herstellt, in der $R^1$ einen tert.Butylrest bedeutet und $R^2$ für Wasserstoff oder Methyl steht.

**4.** Verfahren nach Anspruch 3, dadurch gekennzeichnet, daß man die Verbindung der Formel I mit $R^1$ = tert.Butylrest und $R^2$ = Wasserstoff herstellt ( = 3-(3,5-Di-tert.butyl-4-hydroxyphenyl) -6,7-dihydro-2H-imidazo [2,1-b] [1,3,4]thiadiazin).

**5.** Verfahren zur Herstellung der Verbindungen der Formel I sowie deren physiologisch verträglicher Säureadditionssalze gemäß einem oder mehreren der Ansprüche 1 - 4 zur Anwendung als Heilmittel.

**6.** Verfahren zur Herstellung eines Arzneimittels, dadurch gekennzeichnet, daß man mindestens eine Verbindung der Formel I gemäß der Definition in Anspruch 1 und/oder mindestens ein physiologisch verträgliches Säureadditionssalz einer solchen Verbindung
mit einem physiologisch annehmbaren Träger und gegebenenfalls weiteren Zusatz- und/oder Hilfsstoffen in eine geeignete Darreichungsform bringt.

**7.** Verfahren nach Anspruch 6, dadurch gekennzeichnet, daß das verfahrensgemäß hergestellte Arzneimittel zur Vorbeugung und Behandlung von Krankheiten bestimmt ist, bei denen die therapeutische Anwendung von Entzündungshemmern, Immunmodulatoren, Sauerstoffradikale desaktivierenden Mitteln und/oder Hemmstoffen des durch die 5-Lipoxygenase vermittelten Arachidonsäureabbaus indiziert ist.

**8.** Verfahren nach Anspruch 6 oder 7, dadurch gekennzeichnet, daß das verfahrensgemäß hergestellte Arzneimittel zur Vorbeugung und Behandlung von entzündlichen, insbesondere von entzündlich rheumatischen Erkrankungen bestimmt ist.

**9.** Verwendung von Verbindungen der Formel I gemäß der Definition in Anspruch 1 und/oder von deren physiologisch verträglichen Säureadditionssalzen zur Herstellung von Arzneimitteln zur Vorbeugung und Behandlung der in den Ansprüchen 7 und 8 genannten Krankheiten.

**10.** 1-Amino-2-mercapto-imidazol und dessen tautomere Form der Formeln

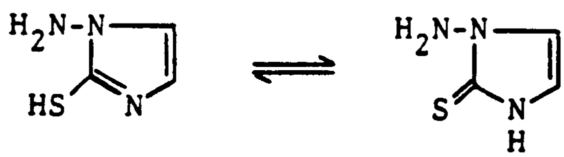

**11.** Verfahren zur Herstellung des 1-Amino-2-mercapto-imidazols gemäß Anspruch 10, dadurch gekennzeichnet, daß man ein Thioisocyanatoacetaldehyddialkylacetal mit Hydrazin umsetzt.

**12.** 1-Amino-2-thioxo-5-imidazolidinon und dessen tautomere Formen der Formeln

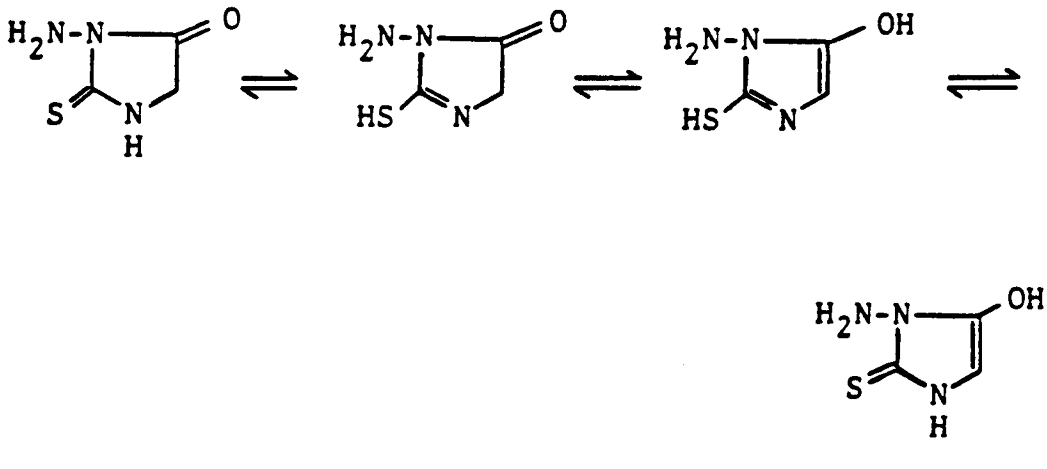

**13.** Verfahren zur Herstellung des 1-Amino-2-thioxo-5-imidazolidinons gemäß Anspruch 12, dadurch gekennzeichnet, daß man einen Thiosemicarbazido-(4)-essigsäurealkylester alkalisch cyclisiert.

**Claims**

**Claims for the following Contracting States : AT, BE, CH, DE, FR, GB, IT, LI, LU, NL, SE**

**1.** An imidazo- or triazolothiadiazine of the formula I

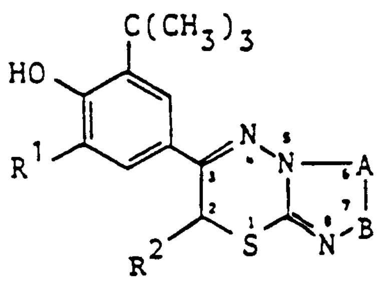

(I)

in which

| | |
|---|---|
| $R^1$ | denotes a straight-chain or branched alkyl group having 1 to 4 carbon atoms, and |
| $R^2$ | denotes a hydrogen atom or an alkyl radical having 1 to 3 carbon atoms, and the twomembered structural element |
| -A-B- | in the five-membered ring represents $-CH_2-CH_2-$, -CH = CH-, -CH = N-, $-CH_2-CO-$ or $-CO-CH_2-$, |

or a physiologically acceptable acid-addition salt thereof.

**2.** A compound as claimed in claim 1, which has at least one of the features that the structural element -A-B- denotes an ethylene or vinylene group, or either A or B represents a carbonyl group and the other bridging member represents a methylene group.

**3.** A compound as claimed in claim 1 or 2, wherein $R^1$ denotes a tert.butyl radical and $R^2$ represents hydrogen or methyl.

**4.** A compound as claimed in claim 3, which is 3-(3,5-di-tert.butyl-4-hydroxyphenyl)-6,7-dihydro-2H-imidazo[2,1-b][1,3,4]thiadiazine.

**5.** A process for the preparation of an imidazo- or triazolothiadiazine of the formula I or a physiologically acceptable acid-addition salt thereof, as claimed in one or more of claims 1 to 4, which comprises reacting a 2-halo-1-phenylalkanone of the formula II

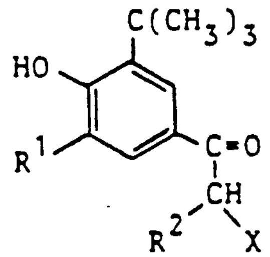

(II)

with a compound of the formula III

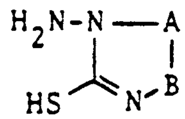

(III)

where $R^1$, $R^2$, and the two-membered structural element -A-B- have the meanings mentioned in claim 1 and X represents a halogen atom, to give a compound of the formula I, and either isolating the latter in free form or converting it into a physiologically acceptable addition salt using a suitable acid.

**6.** A compound of the formula I or a physiologically acceptable acid-addition salt thereof, as claimed in one or more of claims 1 - 4, for use as a medicament.

**7.** A medicament, containing - or comprising - at least one compound of the formula I and/or one of the physiologically acceptable acid-addition salts thereof, as claimed in one or more of claims 1 to 4.

**8.** A medicament as claimed in claim 7, which is intended for prevention and treatment of disorders in which the therapeutic use of inflammation inhibitors, immunomodulators, oxygen radicaldeactivating agents and/or inhibitors of arachidonic acid degradation induced by 5-lipoxygenase is indicated.

**9.** A medicament as claimed in claim 7 or 8, which is used for prevention and treatment of inflammatory, in particular inflammatory rheumatic disorders.

**10.** A process for the production of a medicament as claimed in one or more of claims 7 - 9, which comprises converting at least one compound of the formula I and/or at least one physiologically acceptable acid-addition salt of such a compound into a suitable form of administration together with a physiologically acceptable excipient and, if appropriate, further additives and/or adjuvants.

**11.** The use of a compound of the formula I and/or a physiologically acceptable acid-addition salt thereof for the production of medicaments as claimed in claims 8 and 9.

**12.** 1-Amino-2-mercaptoimidazole and its tautomeric form of the formulae

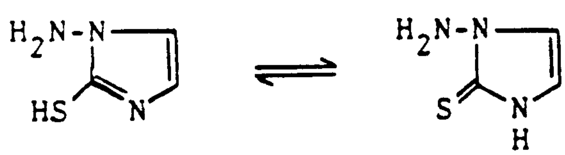

**13.** A process for the preparation of 1-amino-2-mercaptoimidazole as claimed in claim 12, which comprises reacting a thioisocyanatoacetaldehyde dialkyl acetal with hydrazine.

**14.** 1-Amino-2-thioxo-5-imidazolidinone and its tautomeric forms of the formulae

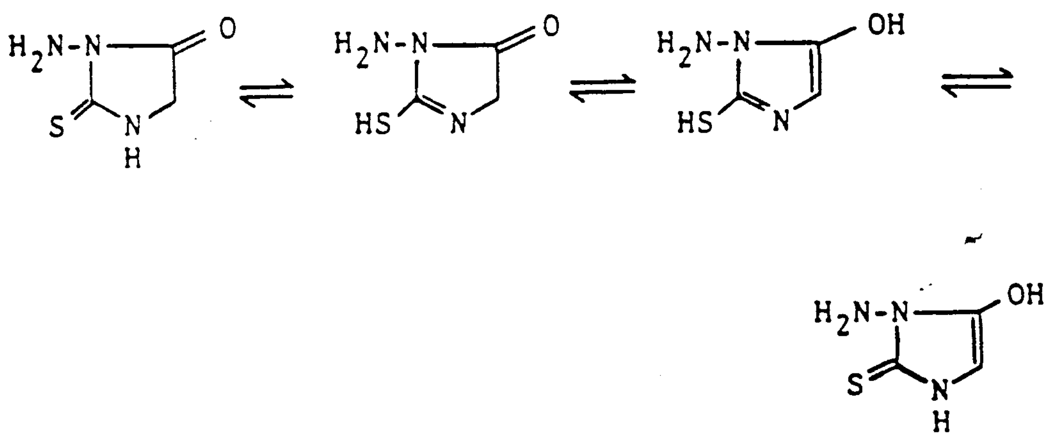

**15.** A process for the preparation of 1-amino-2-thioxo-5-imidazolidinone as claimed in claim 14, which comprises cyclizing an alkyl thiosemicarbazido-(4)-acetate by means of an alkali.

**Claims for the following Contracting States : ES, GR**

**1.** A process for the preparation of imidazo- or triazolothiadiazines of the formula I

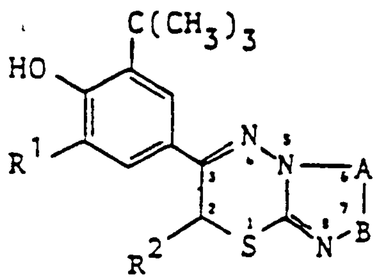

(I)

in which

$R^1$ denotes a straight-chain or branched alkyl group having 1 to 4 carbon atoms, and

$R^2$ denotes a hydrogen atom or an alkyl radical having 1 to 3 carbon atoms, and the twomembered structural element

-A-B- in the five-membered ring represents $-CH_2-CH_2$,$-CH=CH-$, $-CH=N-$, $-CH_2-CO-$ or $-CO-CH_2-$,

or a physiologically acceptable acid-addition salt thereof which comprises reacting a 2-halo-1-phenylalkanone of the formula II

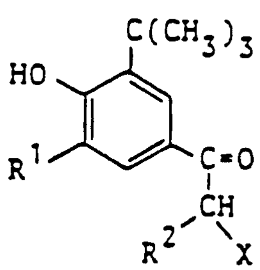

(II)

with a compound of the formula III

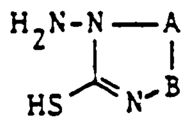

(III)

where $R^1$, $R^2$ and the two-membered structural element -A-B- have the meanings mentioned for formula I and X represents a halogen atom, to give a compound of the formula I, and either isolating the latter in free form or converting it into a physiologically acceptable addition salt by means of a suitable acid.

2. The process as claimed in claim 1, wherein a compound of the formula I is prepared which has at least one of the features that the structural element -A-B- denotes an ethylene or vinylene group, or either A or B represents a carbonyl group and the other bridging member represents a methylene group.

3. The process as claimed in claim 1 or 2, wherein a compound of the formula I is prepared in which $R^1$ denotes a tert.butyl radical and $R^2$ represents hydrogen or methyl.

4. The process as claimed in claim 3, wherein a compound of the formula I is prepared in which $R^1$ denotes the tert.butyl radical and $R^2$ denotes hydrogen, ( = 3-(3,5-di-tert.butyl-4-hydroxyphenyl)-6,7-dihydro-2H-imidazo[2,1-b)[1,3,4]thiadiazine).

5. The process for the preparation of a compound of the formula I, or a physiologically acceptable acid-addition salt thereof, as claimed in one or more of claims 1 - 4, for use as a medicament.

6. A process for the preparation of a medicament, which comprises converting at least one compound of the formula I in accordance with the definition in claim 1 and/or at least one physiologically acceptable acid-addition salt of such a compound into a suitable form of administration together with a physiologically acceptable excipient and, if appropriate, further additives and/or adjuvants.

7. The process as claimed in claim 6, wherein the medicament prepared thereby is intended for prevention and treatment of disorders in which the therapeutic use of inflammation inhibitors, immunomodulators, oxygen radical-deactivating agents and/or inhibitors of arachidonic acid degradation induced by 5-lipoxygenase is indicated.

8. The process as claimed in claim 6 or 7, wherein the medicament prepared thereby is intended for prevention and treatment of inflammatory, in particular inflammatory rheumatic disorders.

9. The use of a compound of the formula I in accordance with the definition in claim 1 and/or of a physiologically acceptable acid-addition salt thereof for the preparation of medicaments for the prevention and treatment of the disorders mentioned in claims 7 and 8.

10. 1-Amino-2-mercaptoimidazole and its tautomeric form of the formulae

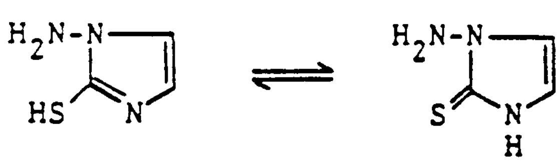

11. A process for the preparation of 1-amino-2-mercapto-imidazole as claimed in claim 10, which comprises reacting a thioisocyanatoacetaldehyde dialkyl acetal with hydrazine.

12. 1-Amino-2-thioxo-5-imidazolidinone and its tautomeric forms of the formulae

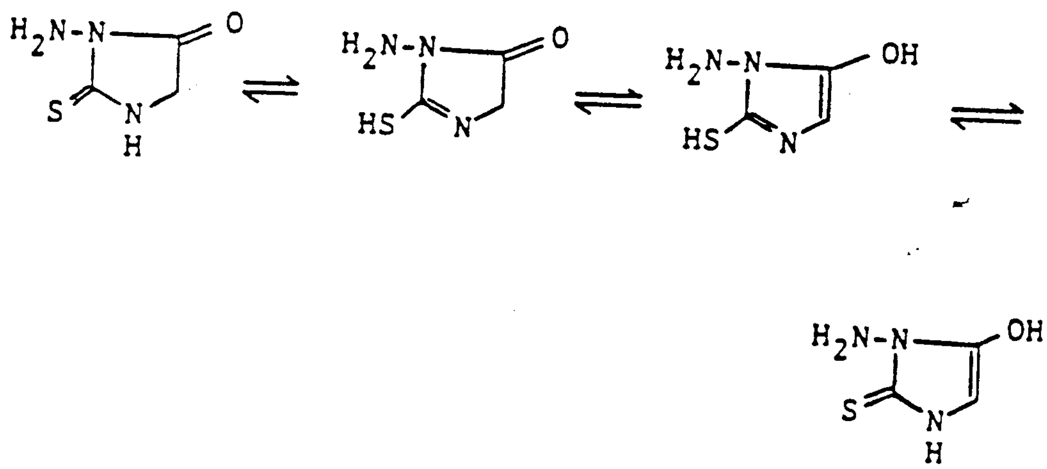

**13.** 13. A process for the preparation of 1-amino-2-thioxo-5-imidazolidinone as claimed in claim 12, which comprises cyclizing an alkyl thiosemicarbazido-(4)-acetate by means of an alkali.

**Revendications**

**Revendications pour les Etats contractants suivants : AT, BE, CH, DE, FR, GB, IT, LI, LU, NL, SE**

**1.** Imidazo- et triazolo-thiadiazines de formule générale I

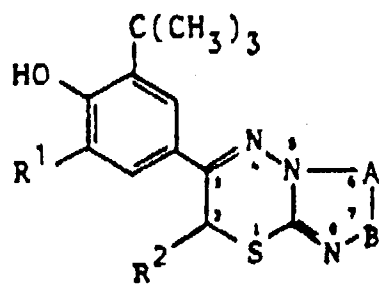

(I),

dans laquelle

$R^1$ représente un groupe alkyle à chaîne droite ou ramifiée ayant de 1 à 4 atomes de carbone et

$R^2$ représente un atome d'hydrogène ou un radical alkyle ayant de 1 à 3 atomes de carbone, et

l'élément structural à deux chaînons -A-B- dans le cycle pentagonal représente $-CH_2-CH_2-$, -CH = CH-, -CH = N-, $-CH_2-CO-$ ou $-CO-CH_2-$,

et sels d'addition avec des acides physiologiquement acceptables de celles-ci.

**2.** Composés selon la revendication 1, caractérisés par au moins l'une des caractéristiques que l'élément structural -A-B- représente un groupe éthylène ou vinylène ou bien soit A, soit B, représente le groupe carbonyle et l'autre chaînon pontant représente le groupe méthylène.

**3.** Composés selon la revendication 1 ou 2, caractérisés en ce que $R^1$ représente le radical tert-butyle et $R^2$ représente un atome d'hydrogène ou le groupe méthyle.

**4.** Composé selon la revendication 3, caractérisé en ce qu'il représente la 3-(3,5-di-tert-butyl-4-hydroxyphényl)-6,7-dihydro-2H-imidazo[2,1-b][1,3,4]thiadiazine.

**5.** Procédé pour la préparation des imidazo- et triazolo-thiadiazines de formule I et de leurs sels d'addition avec des acides physiologiquement acceptables, selon une ou plusieurs des revendications 1 à 4, caractérisé en ce que l'on fait réagir une 2-halogéno-1-phénylalcanone de formule 11

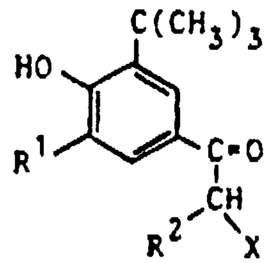

(II)

avec un composé de formule III

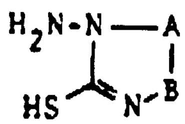

(III)

formules dans lesquelles $R^1$, $R^2$ et l'élément structural à deux chaînons -A-B- a les significations données dans la revendication 1 et X représente un atome d'halogène, pour aboutir aux composés de formule I, et soit on les isole sous forme libre, soit on les convertit, à l'aide d'acides appropriés, en sels d'addition physiologiquement acceptables.

**6.** Composés de formule I et sels d'addition avec des acides physiologiquement acceptables de ceux-ci, selon une ou plusieurs des revendications 1-4, pour utilisation en tant que médicament.

**7.** Médicament, caractérisé par une teneur en - ou consistant en - au moins un composé de formule I et/ou un de ses sels d'addition avec des acides physiologiquement acceptables, selon une ou plusieurs des revendications 1 à 4,

**8.** Médicament selon la revendication 7, caractérisé en ce qu'il est destiné à la prévention ou au traitement de maladies dans lesquelles est indiquée l'application thérapeutique d'anti-inflammatoires, d'immunomodulateurs, d'agents inactivants de radicaux oxygénés et/ou d'inhibiteurs de la dégradation de l'acide arachidonique induite par la 5-lipoxygénase.

**9.** Médicament selon la revendication 7 ou 8, caractérisé en ce qu'il est utilisé pour la prévention et le traitement de maladies inflammatoires, en particulier de maladies inflammatoires rhumatismales.

**10.** Procédé pour la fabrication d'un médicament selon une ou plusieurs des revendications 7 à 9, caractérisé on ce que l'on met sous une forme pharmaceutique appropriée au moins un composé de formule I et/ou au moins un sel d'addition avec un acide physiologiquement acceptable d'un tel composé, avec un véhicule physiologiquement acceptable et éventuellement d'autres additifs et/ou adjuvants.

**11.** Utilisation des composés de formule I et/ou de leurs sels d'addition avec des acides physiologiquement acceptables, pour la fabrication de médicaments selon les revendications 8 et 9.

**12.** 1-amino-2-mercapto-imidazole et sa forme tautomère, de formules

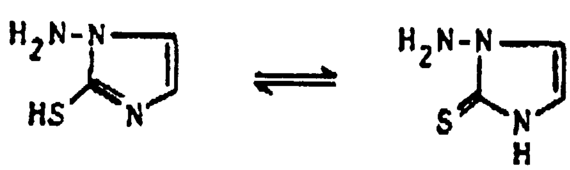

**13.** Procédé pour la préparation du 1-amino-2-mercapto-imidazole selon la revendication 12, caractérisé en

ce que l'on fait réagir un thio-isocyanato-acétaldéhyde-diakyl-acétal avec de l'hydrazine.

**14.** 1-amino-2-thioxo-5-imidazolidinone et ses formes tautomères, de formules

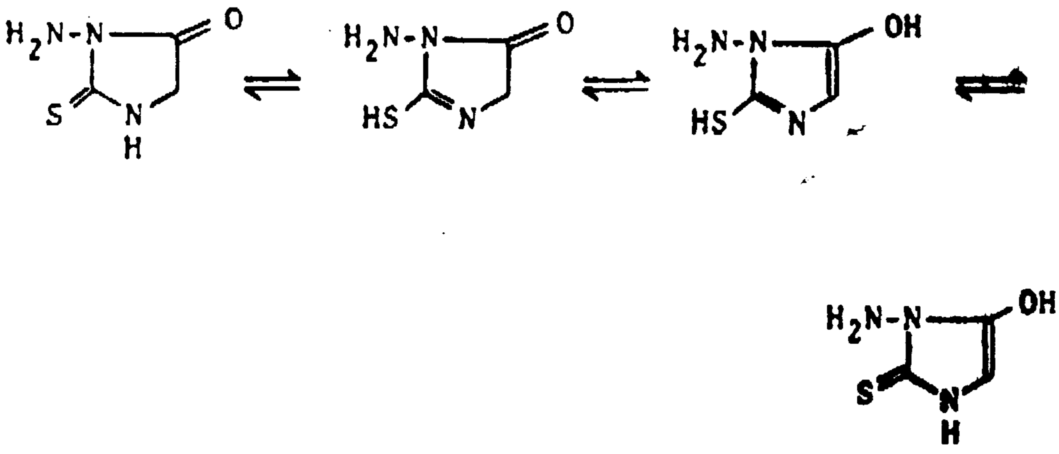

**15.** Procédé pour la préparation de la 1-amino-2-thioxo-5-imidazolidinone selon la revendication 14, caractérisée en ce que l'on soumet à une cyclisation alcaline un thiosemicarbazido-(4)-acétate d'alkyle.

**Revendications pour les Etats contractants suivants : ES, GR**

**1.** Procédé pour la préparation d'imidazo- et triazolothiadiazines de formule générale I

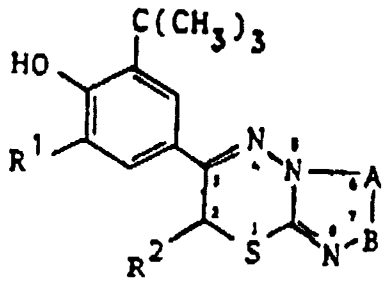

(I)

dans laquelle

$R^1$ représente un groupe alkyle à chaîne droite ou ramifiée ayant de 1 à 4 atomes de carbone et

$R^2$ représente un atome d'hydrogène ou un radical alkyle ayant de 1 à 3 atomes de carbone, et

l'élément structural à deux chaînons -A-B- dans le cycle pentagonal représente $-CH_2-CH_2-$, -CH = CH-, -CH = N-, $-CH_2-CO-$ ou $-CO-CH_2-$,

ainsi que de leurs sels d'addition avec des acides physiologiquement acceptables, caractérisé en ce que l'on fait réagir une 2-halogéno-1-phénylalcanone de formule II

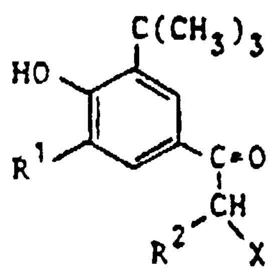

(II)

avec un composé de formule III

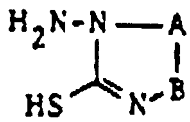

(III)

$R^1$, $R^2$ et l'élément structural à deux chaînons -A-B- ayant les significations données à propos de la formule I et X représentant un atome d'halogène, pour aboutir aux composés de formule I, et soit on les isole sous forme libre, soit on les convertit, à l'aide d'acides appropriés, en sels d'addition physiologiquement acceptables.

**2.** Procédé selon la revendication 1, caractérisé en ce que l'on prépare des composés de formule I ayant au moins l'une des caractéristiques que l'élément structural -A-B-représente un groupe éthylène ou vinylène ou bien soit A, soit B, représente le groupe carbonyle et l'autre chaînon pontant représente le groupe méthylène.

**3.** Procédé selon la revendication 1 ou 2, caractérisé en ce que l'on prépare des composés de formule I dans lesquels $R^1$ représente le radical tert-butyle et $R^2$ représente un atome d'hydrogène ou le groupe méthyle.

**4.** Procédé selon la revendication 3, caractérisé en ce que l'on prépare le composé de formule I dans lequel $R^1$ représente le groupe tert-butyle et $R^2$ représente un atome d'hydrogène ( = 3-(3,5-di-tert-butyl-4-hydroxyphényl)-6,7-dihydro-2H-imidazo[2,1-b][1,3,4]thiadiazine.

**5.** Procédé pour la préparation des composés de formule I ainsi que de leurs sels d'addition avec des acides physiologiquement acceptables, selon une ou plusieurs des revendications 1-4, pour utilisation en tant que médicament.

**6.** Procédé pour la fabrication d'un médicament, caractérisé en ce que l'on met sous une forme pharmaceutique appropriée au moins un composé de formule I selon la définition donnée dans la revendication 1, et/ou au moins un sel d'addition avec un acide physiologiquement acceptable d'un tel composé, avec un véhicule physiologiquement acceptable et éventuellement d'autres additifs et/ou adjuvants.

**7.** Procédé selon la revendication 6, caractérisé en ce que le médicament fabriqué selon le procédé est destiné à la prévention ou au traitement de maladies dans lesquelles est indiquée l'application thérapeutique d'anti-inflammatoires, d'immunomodulateurs, d'agents inactivants de radicaux oxygénés et/ou d'inhibiteurs de la dégradation de l'acide arachidonique induite par la 5-lipoxygénase.

**8.** Procédé selon la revendication 6 ou 7, caractérisé en ce que le médicament fabriqué selon le procédé est destiné à la prévention et au traitement de maladies inflammatoires, en particulier de maladies inflammatoires rhumatismales.

**9.** Utilisation des composés de formule I selon la définition donnée dans la revendication 1 et/ou de leurs sels d'addition avec des acides physiologiquement acceptables, pour la fabrication de médicaments pour la prévention et le traitement des maladies citées dons les revendications 7 et 8.

**10.** 1-amino-2-mercapto-imidazole et sa forme tautomère, de formules

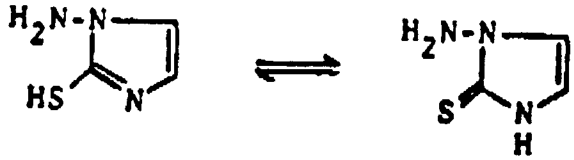

**11.** Procédé pour la préparation du 1-amino-2-mercaptoimidazole selon la revendication 10, caractérisé en ce que l'on fait réagir un thio-isocyanato-acétaldéhyde-dialkyl-acétal avec de l'hydrazine.

**12.** 1-amino-2-thioxo-5-imidazolidinone et ses formes tautomères, de formules

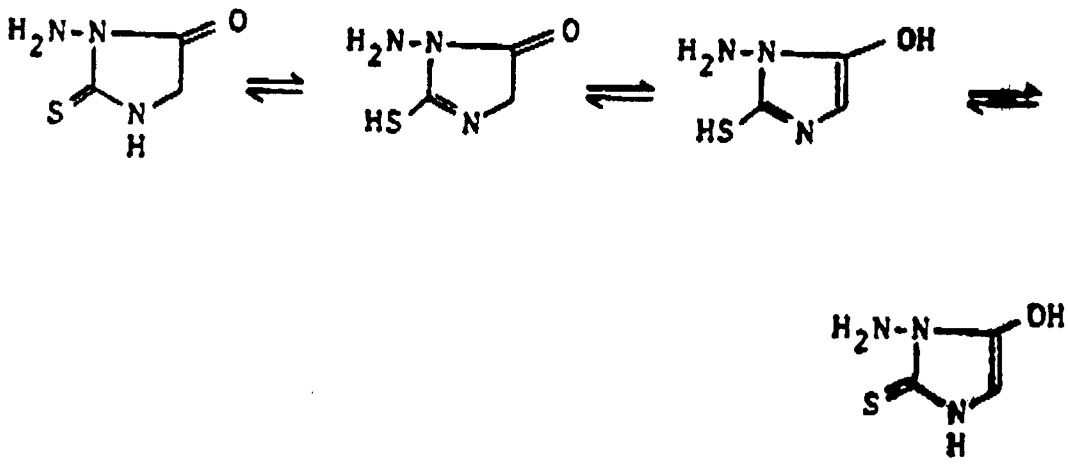

**13.** Procédé pour la préparation de la 1-amino-2-thioxo-5-imidazolidinone selon la revendication 12, caractérisée en ce que l'on soumet à une cyclisation alcaline un thiosemicarbazido-(4)-acétate d'alkyle.